# EUROPEAN PATENT APPLICATION

(11) **EP 1 873 247 A1**
(43) Date of publication of application: **02.01.2008**
(21) Application number: 06116357.2
(22) Date of filing: 29.06.2006
(51) Int. Cl.: C12N 15/54, C12N 15/55, C12N 9/10, C12N 9/16, C12P 19/12

(54) **Novel bifunctional trehalose synthase**

(71) Applicant: VIB vzw, 9052 Zwijnaarde (BE); K.U. Leuven Research and Development, 3000 Leuven (BE)
(72) Inventor: The designation of the inventor has not yet been filed

(57) **Abstract**

The present invention relates to a novel, non-chimeric bifunctional trehalose synthase, comprising both trehalose-6-phophate synthase as well as trehalose-6-phosphate phosphatase activity, as well as to the gene encoding this bifunctional enzyme. The invention relates further to the use of this gene and/or enzyme to increase trehalose accumulation in organisms, especially in bacteria and plants which show no or insufficient trehalose accumulation as wild type.

SEQUENCE LISTING

<110>  VIB vzw
       K.U. Leuven Research and Development

<120>  NOVEL BIFUNCTIONAL TREHALOSE SYNTHASE

<130>  JTH/OtsAB/240

<160>  4

<170>  PatentIn version 3.3

<210>  1
<211>  2202
<212>  DNA
<213>  Cytophaga hutchinsonii

<400>  1
atgaatgacg aaaaaagatt aattattgta gcatatagaa taccttttaa agttacgttt      60

aaagaaagcg gagagcgcgt attacaacaa aattcaggag ggcttgtttc cgctatccta     120

tcattggcag aaaaaggaga tgtgtttggt aaaaatcaaa aaattcattg gtttggttat     180

tccacaccag atattttaca ggagggagaa acgcaggttg aaaatgaaaa ttttattgta     240

catcctgtct taattgaaga tgcactaaat gatgcgtatt acgaaggatt ttgtaacagt     300

acgatttggc ccatttcgca ttattatccc tatctcacca gctttaatga agaggattat     360

gaagcgtatg taaaagcaaa taaaatattt gcagatcaga ttaatgcctt tatccgtccg     420

ggagatatgg tctggataca ggattatcag ctgatgttat tgcctggcat gctccgttca     480

gaaaatccgg atgcttctat cggttatttc catcacatcc cttttgcaac gtttgaaatt     540

tttcgtttgc ttccgaaaca atggcaaaca gaattgttaa aaggtatgat cggcgcagat     600

ctggttggtt ttcataccaa tgattacagc cagtattttt tgagaagtgt gcagcgtgta     660

ttaggctatg atactactgc ccgcgacata agtattccaa acaggatcgt gcgggtagac     720

acatttccca tcagcattga ttataataaa tttcaggaat tgtataatga tccgggcgta     780

attgaaaaaa ggaaagagtt tttaaaagac ctcagtgcag agaaaaatat tttttccgtt     840

gaccggctag attattcaaa aggattgatc catcggttaa atgggtttga atattttctg     900

gatcagtatc cgcaatggca tacacgtgtt gtacttacaa tgattgtagt tccttcgcgt     960

gaaacaatcc tgaagtatgc tgagatgaaa cacgaactgg aagaagcggt aggccgcatt    1020

aacggtaagt acggtacctt aggctggcgt ccggttatat atctgtaccg ttcgttaagc    1080

tttgaagaat taggcgcgtt atatacactg gccgatgtgg cactcatcac gcccatccgt    1140

gacgggatga atttagttgc aaaagaattt gttgctacgc gtaccgataa aaaaggcgta    1200

ttgattttaa gtgaaatggc aggtgcgtcg ctggaattga atggtgcaat actgatcaac    1260

ccaacagaca agaggacact ggccgcagcc attattcagg cattaaccat ggacgagcag    1320

gagcagacac aacgtatgca gatgatgcag gaccggatcc gtgtgtatga tatatttaaa    1380

tggacagaag actacatgga tcaattagat attataaaat tgaaacaaat ggaattcgaa    1440

gagaaaatta taaaccagga tgtgttaaaa agtttcaaac agatttttga tgaatcaagc    1500

cggcgcttgt ttttattgga ttatgacgga accttatctc ccattgttcc tgacccctcc 1560

agttctaagc caagtgtagg tattgttgat gtattaaaaa aactcagcag tgattttaat 1620

gtaaatgttg ctatcataag cggaagagat aaagagtttt tgaattatat ctttgatcca 1680

aatacaattc tgtctgcaga gcatggcgcg ttcttacgta ttccgggtca ggattgggag 1740

ctgcaatatg attacaatga tcagtggaag cgttctgtat tgccggtgtt tcagaagtat 1800

accgatcgtt gcgccggatc ttttattgaa aataaatcaa catcacttgc ctggcattac 1860

agaaatacag aaaaagaata tgctcatatc cgttccagag aatttattga agaactggaa 1920

aacaaagttg ggataaaatc aaatctcgcc attattgatg gtgataaagt aattgaaatg 1980

aaaccttctg atgtagataa ggggattgct gcgcggaaaa tatgtgatct gtatcagccg 2040

gattttattt tatctattgg agatgaccgg actgatgaag acatgtttaa agcgcttccg 2100

gataatgcgc ttacaattaa agttggcgtt aaaaatagtt ctgccaagtt tacaattaaa 2160

acgcaggaag aagtaatgac cattctgaat acgtttttat aa 2202

<210> 2
<211> 733
<212> PRT
<213> Cytophaga hutchinsonii

<400> 2

Met Asn Asp Glu Lys Arg Leu Ile Ile Val Ala Tyr Arg Ile Pro Phe
1               5                   10                  15

Lys Val Thr Phe Lys Glu Ser Gly Glu Arg Val Leu Gln Gln Asn Ser
            20                  25                  30

Gly Gly Leu Val Ser Ala Ile Leu Ser Leu Ala Glu Lys Gly Asp Val
        35                  40                  45

Phe Gly Lys Asn Gln Lys Ile His Trp Phe Gly Tyr Ser Thr Pro Asp
    50                  55                  60

Ile Leu Gln Glu Gly Glu Thr Gln Val Glu Asn Glu Asn Phe Ile Val
65                  70                  75                  80

His Pro Val Leu Ile Glu Asp Ala Leu Asn Asp Ala Tyr Tyr Glu Gly
                85                  90                  95

Phe Cys Asn Ser Thr Ile Trp Pro Ile Ser His Tyr Tyr Pro Tyr Leu
            100                 105                 110

Thr Ser Phe Asn Glu Glu Asp Tyr Glu Ala Tyr Val Lys Ala Asn Lys
        115                 120                 125

Ile Phe Ala Asp Gln Ile Asn Ala Phe Ile Arg Pro Gly Asp Met Val
    130                 135                 140

Trp Ile Gln Asp Tyr Gln Leu Met Leu Leu Pro Gly Met Leu Arg Ser
145                 150                 155                 160

Glu Asn Pro Asp Ala Ser Ile Gly Tyr Phe His His Ile Pro Phe Ala
                165                 170                 175

Thr Phe Glu Ile Phe Arg Leu Leu Pro Lys Gln Trp Gln Thr Glu Leu
            180                 185                 190

Leu Lys Gly Met Ile Gly Ala Asp Leu Val Gly Phe His Thr Asn Asp
        195                 200                 205

Tyr Ser Gln Tyr Phe Leu Arg Ser Val Gln Arg Val Leu Gly Tyr Asp
    210                 215                 220

Thr Thr Ala Arg Asp Ile Ser Ile Pro Asn Arg Ile Val Arg Val Asp
225                 230                 235                 240

Thr Phe Pro Ile Ser Ile Asp Tyr Asn Lys Phe Gln Glu Leu Tyr Asn
                245                 250                 255

Asp Pro Gly Val Ile Glu Lys Arg Lys Glu Phe Leu Lys Asp Leu Ser
            260                 265                 270

Ala Glu Lys Asn Ile Phe Ser Val Asp Arg Leu Asp Tyr Ser Lys Gly
        275                 280                 285

Leu Ile His Arg Leu Asn Gly Phe Glu Tyr Phe Leu Asp Gln Tyr Pro
    290                 295                 300

Gln Trp His Thr Arg Val Val Leu Thr Met Ile Val Val Pro Ser Arg
305                 310                 315                 320

Glu Thr Ile Leu Lys Tyr Ala Glu Met Lys His Glu Leu Glu Glu Ala
                325                 330                 335

Val Gly Arg Ile Asn Gly Lys Tyr Gly Thr Leu Gly Trp Arg Pro Val
            340                 345                 350

Ile Tyr Leu Tyr Arg Ser Leu Ser Phe Glu Glu Leu Gly Ala Leu Tyr
        355                 360                 365

Thr Leu Ala Asp Val Ala Leu Ile Thr Pro Ile Arg Asp Gly Met Asn
    370                 375                 380

Leu Val Ala Lys Glu Phe Val Ala Thr Arg Thr Asp Lys Lys Gly Val
385                 390                 395                 400

Leu Ile Leu Ser Glu Met Ala Gly Ala Ser Leu Glu Leu Asn Gly Ala
                405                 410                 415

Ile Leu Ile Asn Pro Thr Asp Lys Arg Thr Leu Ala Ala Ala Ile Ile
            420                 425                 430

Gln Ala Leu Thr Met Asp Glu Gln Glu Gln Thr Gln Arg Met Gln Met
        435                 440                 445

Met Gln Asp Arg Ile Arg Val Tyr Asp Ile Phe Lys Trp Thr Glu Asp
    450                 455                 460

Tyr Met Asp Gln Leu Asp Ile Ile Lys Leu Lys Gln Met Glu Phe Glu
465                 470                 475                 480

Glu Lys Ile Ile Asn Gln Asp Val Leu Lys Ser Phe Lys Gln Ile Phe
                485                 490                 495

Asp Glu Ser Ser Arg Arg Leu Phe Leu Leu Asp Tyr Asp Gly Thr Leu
            500                 505                 510

Ser Pro Ile Val Pro Asp Pro Ser Ser Ser Lys Pro Ser Val Gly Ile
        515                 520                 525

Val Asp Val Leu Lys Lys Leu Ser Ser Asp Phe Asn Val Asn Val Ala
    530                 535                 540

Ile Ile Ser Gly Arg Asp Lys Glu Phe Leu Asn Tyr Ile Phe Asp Pro
545                 550                 555                 560

Asn Thr Ile Leu Ser Ala Glu His Gly Ala Phe Leu Arg Ile Pro Gly
                565                 570                 575

Gln Asp Trp Glu Leu Gln Tyr Asp Tyr Asn Asp Gln Trp Lys Arg Ser
            580                 585                 590

Val Leu Pro Val Phe Gln Lys Tyr Thr Asp Arg Cys Ala Gly Ser Phe
        595                 600                 605

Ile Glu Asn Lys Ser Thr Ser Leu Ala Trp His Tyr Arg Asn Thr Glu
    610                 615                 620

Lys Glu Tyr Ala His Ile Arg Ser Arg Glu Phe Ile Glu Glu Leu Glu
625                 630                 635                 640

Asn Lys Val Gly Ile Lys Ser Asn Leu Ala Ile Ile Asp Gly Asp Lys
                645                 650                 655

Val Ile Glu Met Lys Pro Ser Asp Val Asp Lys Gly Ile Ala Ala Arg
            660                 665                 670

Lys Ile Cys Asp Leu Tyr Gln Pro Asp Phe Ile Leu Ser Ile Gly Asp
        675                 680                 685

Asp Arg Thr Asp Glu Asp Met Phe Lys Ala Leu Pro Asp Asn Ala Leu
    690                 695                 700

Thr Ile Lys Val Gly Val Lys Asn Ser Ser Ala Lys Phe Thr Ile Lys
705                 710                 715                 720

Thr Gln Glu Glu Val Met Thr Ile Leu Asn Thr Phe Leu
                725                 730

<210>  3
<211>  32
<212>  DNA
<213>  Artificial

<220>
<223>  Forward primer

<400>  3
ggcctcgaga tgaatgacga aaaagattaa tt                                32

<210>  4
<211>  29
<212>  DNA
<213>  Artificial

<220>
<223>  Reverse primer

<400>  4
ggcgagctca taaaacgtat tcagaatgg                                    29

## Description

The present invention relates to a novel, non-chimeric bifunctional trehalose synthase, comprising both trehalose-6-phophate synthase as well as trehalose-6-phosphate phosphatase activity, as well as to the gene encoding this bifunctional enzyme. The invention relates further to the use of this gene and/or enzyme to increase trehalose accumulation in organisms, especially in bacteria and plants which show no or insufficient trehalose accumulation as wild type.

One of the most fundamental challenges for cell survival, both in free living state or in tissues, is to keep turgor in response to changes in the environment, such as extreme temperatures, salinity, or dehydration. This problem was to be solved very early in cell evolution since the first organisms inhabited the primitive seas (Cavalier-Smith, 2001; McMullan *et al.,* 2004). During evolution, organisms have developed two different strategies to contend with this problem. In certain organisms that live in extreme environments, as strict thermophyles and halophyles, the metabolic capabilities were modified, such that the optimal activity of their enzymes are at high temperature or salinity, respectively (Madigan and Oren, 1999). On the other hand, several organisms exposed to unfavorable conditions evolved a drastically different strategy to contend with the stress.

They developed new biosynthetic pathways for osmotically active compounds, which diminish the osmotic effects without compromising the cellular metabolism. Among these compounds are certain type of polyols as mannitol, sorbitol, and some amino acids as proline and glutamic acid; quaternary ammonium salts, as glycine betaine, and disaccharides as sucrose and trehalose (Yancey *et al.,* 1982). The latter compound is a non-reducing disaccharide formed by two glucose molecules linked by 1α-1α bounds which is present in several organisms and food stuffs, as bread, beer, vinegar, and honey (Elbein *et al.,* 2003).

Many functions have been described for trehalose: in prokaryotes *in vivo* synthesized trehalose is frequently used as a solute compatible to contend with osmotic stress. Also, trehalose can be used as an external carbon source (Strøm and Kaasen, 1993; Horlacher and Boss, 1997). In bacteria of the genus *Mycobacteria, Nocardia, Rhodococcus* and

*Corynebacterium,* trehalose is present in the cell wall glycolipids (Argüelles, 2000; De Smet *et al.,* 2000; Richards *et al.,* 2002). In fungi, as yeast, trehalose can be used as a reserve compound (Elbein, 1974; Thevelein, 1984) and as pivotal molecule relevant for the adaptive response to different types of stresses, such as osmotic, extreme temperature, dehydration, and oxidative (Argüelles, 2000; Hounsa *et al.,* 1988; De Virgillio *et al.,* 1994; Hottiger *et al.,* 1994; Singer and Lindquist, 1988). Also, it has been shown that in yeast the trehalose biosynthesis intermediary trehalose 6-phosphate is a regulator of the glucose metabolic flux during glycolysis (Thevelein and Hohmann, 1995; Neves *et al.,* 1995; Hohmann *et al.,* 1996; Gancedo and Flores, 2004).

In several organisms trehalose is capable of stabilizing and protecting cell structures, as membranes and proteins, allowing anhydrobiotic organisms to survive cycles of dehydration-rehydration (Weisburd, 1988; Colaço *et al.,* 1992). In insects trehalose is used as carbon reserve for energy; it is the most abundant sugar in the hemolymph (80-90%) and in the thorax muscles, were it is consumed during flight (Richards *et al.,* 2002; Becker *et al.,* 1996). Until recently, it was thought that in plants trehalose was only synthesized in the so-called "resurrection" plants as *Selaginella lepidophylla* and *Myrothammus flabellifolius,* where it is the key molecule to protect against stress, especially drought. However, a large number of studies with transgenic plants and the sequencing information available for many plant genomes have shown that trehalose can be synthesized in several other plants. Furthermore, in *Arabidopsis thaliana* it has been demonstrated that trehalose has a fundamental role in embryo development (Eastmond *et al.* 2002), and in abscisic acid and sugar signaling (Avonce *et al., 2004).*

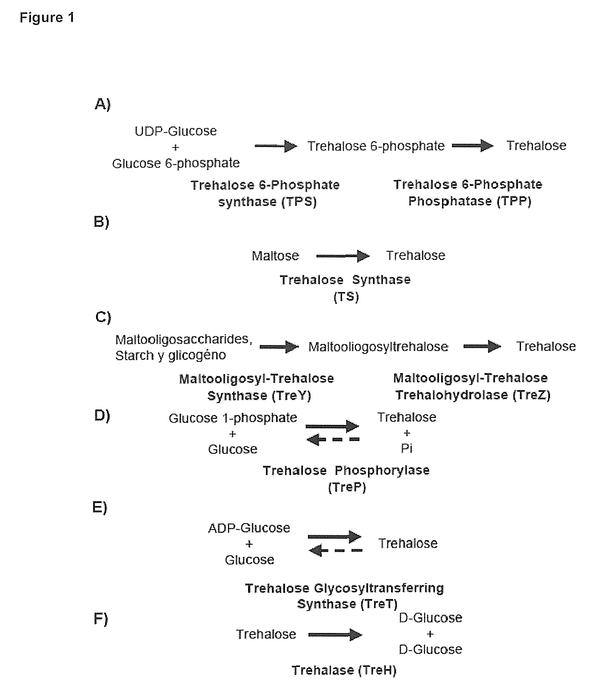

There are at least five biosynthetic pathways known for trehalose (Figure 1). The first pathway reported about 50 years ago (Cabib and Lenoir, 1958), is the most widely distributed, and it has been reported in eubacteria, archea, fungi, insects, and plants. It involves two enzymatic steps catalyzed by trehalose-6-phosphate synthase (TPS) and trehalose-6-phosphate phosphatase (TPP). TPS catalyzes the transfer of glucose from UDP-glucose to glucose 6-phosphate forming trehalose 6-phosphate (T6P) and UDP, while TPP dephosphorylates T6P to trehalose and inorganic phosphate (Elbein *et al.,* 2003; De Smet *et al.,* 2000) (Figure 1A). In the second biosynthetic pathway the enzyme trehalose synthase (TS) isomerizes the α1-α4 bond of maltose to an α1-α1 bond, forming trehalose. This enzyme was first reported in *Pimelobacter sp,* and orthologs of this protein have been found in other eubacteria, insects, nematodes (Elbein *et al.,* 2003; Higashiyama, 2002) and mammals (Figure 1B). The third pathway involves the maltodextrines (maltooligosaccharides, glycogen and starch) conversion in trehalose. This pathway was reported in thermophylic archeas of the genus *Sulfolobus.* These organisms synthesize trehalose in two enzymatic steps catalyzed by maltooligosyl trehalose synthase (MTS), which promotes the transglycosylation of the last glucose moiety at the reducing end of maltodextrins from a α1-α4 to a α1-α1 bond leading to maltooligosyltrehalose, which contains a trehalose moiety at the end of the polymer. Subsequently, the enzyme maltooligosyl trehalose trehalohydrolase (MTH) catalyzes the hydrolysis of trehalose (Figure 1C) (Elbein *et al.* 2003; Streeter and Bhagwat, 1999). In the fourth pathway the enzyme trehalose phosphorylase (TREP), present in some fungi, catalyzes the reversible hydrolysis of trehalose in the presence of inorganic phosphate. The transfer of a glucose molecule to a phosphate generates glucose 1-phosphate and releases the other glucose residue. There is discrepancy about the participation of the TREP enzyme in the synthesis or degradation of trehalose, since the biosynthetic reaction has only been shown *in vitro* (Wannet *et al.,* 1998; Schiraldi *et al.,* 2002) (Figure 1D). Recently, it was found in *Thermococcus litoralis,* a hyperthermophylic archea, a new biosynthetic pathway for trehalose. The enzyme trehalose glicosyltransferring synthase (TRET) catalyzes the reversible formation of trehalose from ADP-glucose and glucose. It can also use UDPglucose and GDP- glucose, although with these substrates it is less efficient. The TRET enzyme transfers the glucose moiety from ADP-glucose, and binds to it at position 1 of the other glucose molecule to form trehalose (Qu *et al.,* 2004) (Figure 1 E).

Although, as described above, trehalose accumulation is a rather generally occurring phenomenon, there are several organisms that show no or insufficient trehalose accumulation and that would benefit from trehalose accumulation to increase their stress resistence. WO2006018446 describes that improved trehalose accumulation in lactic acid bacteria such as *Lactococcus lactis* leads to a better survival after freeze drying and to an improved tolerance to acid and bile salts. WO 2004000008 describes that increased trehalose accumulation in monocots results in an increased resistance against abiotic stress. Even in organisms, where there is already a significant trehalose accumulation, such as in baker's yeast *(Saccharomyces cerevisiae),* further accumulation of trehalose may lead to improved storage characteristics.

Trehalose accumulation in organisms with no, or insufficient trehalose accumulation is normally obtained by transformation of the host organism with both trehalose-6-phosphate synthase and trehalose-6-phosphate phosphatase. As a non-limiting example, transformation and expression of the *Escherichia coli* OtsA and OtsB genes can be used. Alternatively, the *Saccharomyces cerevisiae* genes may be used. However, although the *coli* genes are expressed in one operon, and the yeast enzyme works in one multisubunit complex, it is not evident to obtain a balanced expression of both genes, and the trehalose accumulation might not be optimal. To overcome this problem, Seo *et al.* (2000) made a chimeric, bifunctional fusion enzyme by fusing the *E. coli* genes for trehalose-6-phosphate synthase and trehalose-6-phosphate phosphatase into one chimeric construct. They demonstrated that the bifunctional fusion enzyme has kinetic advantages over a mixture of both enzymes.

Although this construct is an improvement above the mixture of two enzymes, it might still be suboptimal, as the fusion of both enzymes is likely to give a distortion of the enzymatic active centers. Optimalisation of the chimeric construct would require full insight in the structure function relation, and advanced modeling to improve the chimeric construct.

There are several plant TPS genes that encode both a synthethase and a phosphates domain (so called class II TPS; Leyman *et al.,* 2001; Eastman *et al.,* 2003) but up till now, no protein was isolated showing both activities. Indeed, several of the TPS genes of *Arabidopsis* have been tested in yeast, but they were only complementing the synthase function (Vogel *et al.,* 2001).

Surprisingly, we found now that the trehalose synthase gene from *Cytophaga hutchinsonii* shows both synthase and phosphatase activities and can complement both functions in yeast.

A first aspect of the invention is a non-chimeric trehalose synthase, comprising at least trehalose-6-phosphate synthase and trehalose-6-phosphate phosphatase activity (abbreviated as TPSP). Non-chimeric as used here means that all domains, essential for the activity of the protein are originating from one protein, i.e. the resulting trehalose synthase is not a fusion protein consisting of a trehalose-6-phosphate synthase domain of one protein and a trehalose-6-phosphate phosphatase domain of another protein. As a non-limiting example, this could be realized by mutational activation of a non active TPP domain in an *Arabidopsis thaliana* class II TPS. Alternatively, such protein and/or the gene encoding it may be isolated from a plant, fungal, algae, archaea or bacterial species. Preferably, said non-chimeric trehalose synthase is isolated from *Cytophaga hutchinsonii, Anaeromyxobacter dehalogenans, Croscosphera watsonii* or *Pyrobaculum aerophilum.* More preferably, said non-chimeric trehalose synthase is isolated from *Cytophaga hutchinsonii.* Most preferably, said non-chimeric trehalose synthase comprises, preferably consists of SEQ ID N° 2.

Another aspect of the invention is a gene, encoding a non-chimeric trehalose synthase according to the invention. Preferably, the coding sequence of said gene comprises the coding sequence of SEQ ID N°1, even more preferably it consist of the coding sequence of SEQ ID N°1.

Still another aspect of the invention is a method to obtain trehalose accumulation in an organism, comprising overexpression of a non-chimeric trehalose synthase according to the invention. Overexpression, as used here, means that the amount of messenger RNA and/or the activity of TPS and TPP in the overexpressed situation is higher than in the control situation. Overexpression according this definition can be obtained both in organism in which the gene according to the invention is expressed in a natural situation, as in organism wherein said gene is not expressed or not present. Methods for overexpression are known to the person skilled in the art and include, but are not limited to placing a trehalose synthase gene according to the invention under the control of a strong promoter, duplicating or multiplying the numbers of copies of the gene expressed, of transformation of the gene into an organism wherein the gene originally was not present. Said organism may be any organism, including but not limited to animals, insects, plants and micro-organisms. Preferably, said organism is selected from the group consisting of plants, bacteria, and fungi (including yeasts). In one preferred embodiment, said organism is a plant. In another preferred embodiment, said organism is *Saccharomyces cerevisiae.* In still another preferred embodiment, said organism is a lactic acid bacterium, preferably *Lactococcus lactis.*

### BRIEF DESCRIPTION OF THE FIGURES

Figure 1: overview of trehalose synthesis pathways in different organisms.
Figure 2: complementation of the S. *cerevisiae tps1Δtps2Δ* double mutant by overexpression of the C. *hutchinsonii* gene.

### EXAMPLES

Materials and methods to the examples

BLASTP (http://www.ncbi.nih.gov/BLAST/) was used to detect protein sequences in microbial genomes that are homologous to E.coli OtsA (GI:16129848) and OtsB (GI:16129849). The selected sequences were analyzed by the SMART server (http://smart.embl-heidelberg.de/) to determine the domain structure. Specific primers were designed to amplify the ChTPSP gene from the *C.hutchisonii* genomic DNA (ATCC33406D), and new restriction sites were inserted into the primers *(Xho I* and Sac, 5'-3', respectively). The PCR product was cloned in the pSal4 vector (Mascorro-Gallardo *et al.,* 1996; Zentella *et al.,* 2000) using standard protocols, resulting in the pSal4ChTPSP plasmid.

To evaluate the functionality of the ChTPSP gene, the pSal4ChTPSP plasmid was transformed into the *tps1Δtps2Δ*-yeast mutant strain using the Gietz method (Gietz *et al.,* 1995) and phenotypical complementation was analyzed on SDGlucose-URA plates.

To determine TPS and TPP activity, and trehalose accumulation, 50ml cultures were grown to stationary phase, protein extracts were prepared and enzymatic activities were assayed according to the standard protocols (Bell *et al.,* 1998).

### Example 1: identification of candidate TPSP genes.

BLAST searches through the many bacterial and archaea genomes resulted in the identification of the following species that have only one gene in their genome showing homology with both OtsA and OtsB
*Cytophaga hutchinsonii*
*Anaeromyxobacter dehalogenans* (one duplicated gene)
*Croscosphera watsonii*
*Pyrobaculum aerophilum*

### Example 2: cloning of the Cytophaga hutchinsonii TPSP gene

DNA from *Cytophaga hutchinsonii* was obtained from ATCC. Two primers were designed to isolate the gene.
Forward primer: 5' GGCCTCGAGATGAATGACGAAAAAGATTAATT 3'
Reverse primer: 5' GGCGAGCTCATAAAACGTATTCAGAATGG 3'
The PCR product with a length of 2215 pb was cloned in the yeast expression vector pSal4, using the restriction sites Xhol and *Sac*I, resulting in pSAL4ChTPSP.

### Example 3: yeast complementation assay and TPS / TPP activity measurement

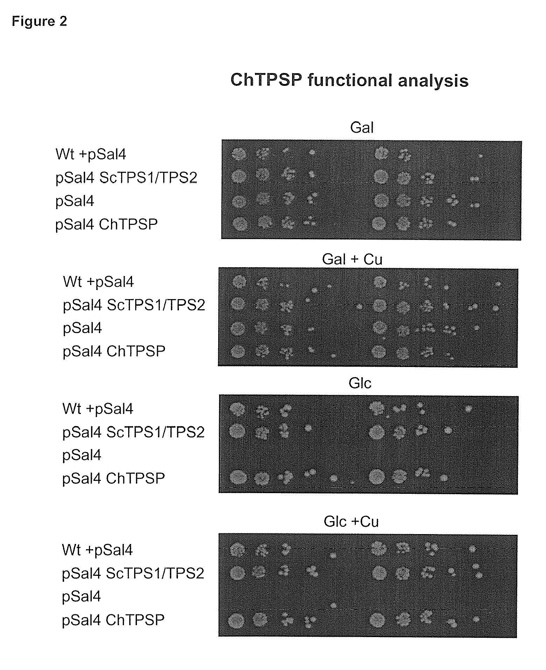

pSAL4ChTPSP was transformed to the yeast *tps1*Δ*tps2*Δ strain and plated on SDglucose-ura plates. The yeast transformants were tested for their capacity to complement the glucose sensitivity of the yeast *tps1*Δ*tps2*Δ mutant. The result is shown in Fig. 2. It is clear that the C. *hutchinsonii* TPSP enzyme can complement the growth defect of the yeast *tps1*Δ mutant, even without the inducer copper sulfate.
To measure the TPS and TPP activity, the yeast transformants were grown till stationary phase, extracts were prepared and trehalose-6-phosphate synthase, trehalose-6-phosphate phosphatase, as well as trehalose levels were determined. Table 1 shows the results of these measurements. For comparison we have included the yeast wild type strain and a strain in which a functional yeast hybrid construct was introduced. The results are the mean of two independent experiments

**Table1.**

| Strain | TPS Activity (µKat/g protein) | TPP Activity (nKat/g protein) | Trehalose (µmol/g ww) |
|---|---|---|---|
| Wt pSal4 | 3.9 | 212,3 | 48.7 |
| *tps1*Δ *tps2*Δ + pSAL4 | 0.1 | -29,1 | 4.3 |
| *tps1*Δ *tps2*Δ *+* pSal4 ScTPS1-TPS2 | 11.9 | 3137,3 | 35.8 |
| *tps1*Δ *tps2*Δ + pSal4 ChTPSP | 5.1 | 1289 | 38.5 |

### REFERENCES

- Argüelles, J.C. (2000) Physiological roles of trehalose in bacteria and yeast: a comparative analysis. Arch Microbiol, 174: 217-224.
- Avonce, N., Leyman, B., Mascorro-Gallardo, O., Van Dijck, P., Thevelein, J.M. and Iturriaga, G. (2004) The Arabidopsis Trehalose-6-P Synthase AtTPS1 Gene Is a Regulator of Glucose, Abscisic Acid, and Stress Signaling. Plant Physiol, 136:3649-59.
- Becker, A., Schloeder, P., Steele, J.E. and Wegener, G. (1996) The regulation of trehalose metabolism in insects. Experientia, 52:433-439.
- Bell, W., Sun, W., Hohmann, S., Wera, S., Reinders, A., De Virgilio, C., Wiemken, A., Thevelein, J.M. (1998) Composition and functional analysis of the Saccharomyces cerevisiae trehalose synthase complex. J Biol Chem 273(50):33311-9.
- Cabib, E. and Lenoir, L.F. (1958) The biosynthesis of trehalose phosphate. J Biol Chem, 231: 259-275.
- Cavalier-Smith, T. (2001) Obscells as proto-organisms: membrane heredity, lithophosphorylation, and the origins of the genetic code, the first cells and photosynthesis. J Mol Evol , 53:555-595.
- Colaço, C., Sen, S., Thangavelu, M., Pinder, S. and Roser, B. (1992) Extraordinary stability of Enzymes dried in trehalose: Simplified molecular biology. Biotechnology, 10:1007-1011.
- De Smet, K.A.L., Weston, A., Brown, I.N., Young, D.B. and Robertson, B.D. (2000) Three pathways for trehalose biosynthesis in mycobacteria. Microbiology, 144:199-208.
- De Virgilio, C., Hottiger, T., Dominguez, J., Boller, T. and Wiemken, A. (1994) The role of trehalose synthesis for the adquisition of thermotolerance in yeast. I. Genetic evidence that trehalose is a thermoprotectant. FEBS, 219:179-186.
- Eastmond, P.J., van Dijken, A.J.H., Spielman, M., Kerr, A., Tissier, A.F., Dickinson, H.G., Jones, J.D.G., Smeekens, S.C. and Graham, LA. (2002) Trehalose-6-phosphate synthase 1, which catalyses the first step in trehalose synthesis, is essential for Arabidopsis embryo maturation. Plant J 2002, 29:225-235.
- Eastman, P.J., Li, Y and Graham, LA. (2003) Is trehalose-6-phosphate a regulator of sugar metabolism in plants? J. Exp. Botany 54, 533-537.
- Elbein, A.D. (1974) The metabolism of α, α-trehalose. Adv Carbohydr Chem Biochem, 30: 227-256.
- Elbein, A.D., Pan, Y.T., Pastuszak, I. and Carroll, D. (2003) New insights on trehalose: a multifuntional molecule. Glycobiology, 13:17R-27R.
- Gancedo, C., Flores, C.L. (2004) The importance of a functional trehalose biosynthetic pathway for the life of yeast and fungi. FEMS Yeast Res, 4: 351-359.
- Gietz, R.D., Schiestl, R.H. (1995) Transforming yeast with DNA. Methods in Molecular and Cell Biology. 5:255-269.
- Higashiyama, T. (2002) Novel functions and applications of trehalose. Pure Appl Chem, 74: 1263-1269.
- Hohmann, S., Bell, W., Neves, M.J., Valckx, D. and Thevelein, J.M. (1996) Evidence for trehalose-6- phosphate-dependent and -independent mechanisms in the control of sugar influx into yeast glycolysis. Mol Microbiol, 20: 981-991.
- Horlacher, R. and Boss, W. (1997) Characterization of TreR, the major regulator of the Escherichia coli trehalose system. J Biol Chem, 272: 13026-13032.
- Hottiger, T., De Virgilio, C., Hall, N.M., Boller, T., Wiemken, A. (1994) The role of trehalose synthesis for the adquisition of thermotolerance in yeast. II. Physiologycal concentrations of trehalose increase the thermal stability of proteins in vitro. FEBS, 219: 187-193.
- Hounsa, C.G., Brandt, E.V., Thevelein, J., Hohmann, S. and Prior, B.A. (1988) Role of trehalose in survival of Saccharomyces cerevisiae under osmotic stress. Microbiology, 144: 671-680.
- Leyman, B., Van Dijck, P., and Thevelein, J.M. (2001) An unexpected plethora of trehalose biosynthesis genes in Arabidopsis thaliana. Trends in Plant Science, 6, 510-513.
- Madigan, M.T. and Oren, A. (1999) Thermophilic and halophilic extremophiles. Curr Opin Microbiol, 2:265-269.
- Mascorro-Gallardo, J.O., Covarrubias, A.A. and Gaxiola, R. (1996) Construction of a CUP1 promoter-based vector to modulate gene expresión in Saccharomyces cerevisiae. Gene 172:169-170.
- McMullan, G., Christie, J.M., Rahman, T.J., Banat, I.M., Ternan, N.G. and Marchant, R. (2004) Habitat, applications and genomics of the aerobic, thermophilic genus Gaobacillus. Biochem Soc Trans, 32:214-217.
- Neves, M.J., Hohmann, S., Bell, W., Dumortier, F., Luyten, K., Ramos, J., Cobbaert, P., de Koning, W., Kaneza, Z. and Thevelein, J.M. (1995) Control of glucose influx into glycolysis and pleiotropic effects studied in different isogenic sets of Saccharomyces cerevisiae mutants in trehalose biosynthesis. Curr Genet, 27: 110-122.
- Qu, Q., Lee, S.J. and Boss, W. (2004) TreT, a novel trehalose glycosyltransferring synthase of the hyperthermophilic archeon Thermococcus litoralis. J Biol Chem, 279:47890-47897.
- Richards, A.B., Krakowka, S., Dexter, L.B., Schmid, H., Wolterbeek, A.P.M., Waalkens-Berendsen, D.H., Shigoyuki, A. and Kurimoto, M. (2002) Trehalose: a review of properties, history of use and human tolerance, and results of multiple safety studies. Food Chem Toxicol, 40: 871-898.
- Schiraldi, C., Di Lernia, I. and De Rosa, M. (2002) Trehalose production: exploiting novel approaches. TRENDS Biotechno, 20: 420-425.
- Seo, H.S., Koo, Y.J., Lim, J.Y., Song, J.T., Kim, C.H., Kim, J.K., Lee, J.S. and Choi, Y.D. (2000) Characterization of a bifunctional enzyme fusion of trehalose-6-phosphatase synthetase and trehalose-6-phosphate phosphatase of Escherichia coli. Appl. Environ. Microbiol 66,2484-2490.
- Singer, M.A. and Lindquist, S. (1988) Thermotolerance in Saccharomyces cerevisiae: the Yin and Yang of trehalose. TIBTECH, 16: 460-468.
- Streeter, J.G. and Bhagwat, A. (1999) Biosynthesis of trehalose from maltooligosaccharides in Rhizobia. Can J Microbiol, 45:716-721. 29.
- Strom, A.R. and Kaasen, I. (1993) Trehalose metabolism in Escherichia coli: stress protection and stress regulation of gene expression. Mol Microbiol, 8:205-210. 10.
- Thevelein, J.M. (1984) Regulation of trehalose mobilization in fungi. Microbiol Rev, 48: 42-59.
- Thevelein, J.M. and Hohmann, S. (1995) Trehalose synthase: guard to the gate of glycolysis in yeast? TIBS, 20:3-10.
- Vogel, G., Fiehn, O., Jean-Richard-dit-Bressel, J., Boller, T., Wiemken, A., Aeschbacher, R.A. and Wingler, A. (2001). Trehalose metabolism in Arabidopsis: occurence of trehalose and molecular cloning and characterization of trehalose-6-phosphate homologues. J. Exp. Botany, 52, 1817-1826.
- Wannet, W.J.B., Op den Camp, H.J.M., Wisselink, H.W., van der Drift, C., Van Griensven, L.J.L.D. and Vogels, G.D. (1998) Purification and characterization of trehalose phosphorylase from the commercial mushroom Agaricus bisporus. Biochim Biophys Acta, 1425:177- 188.
- Weisburd, S. (1988) Death-Defying Dehydration. Sci News, 13: 107-110.
- Yancey, P.H., Clark, M.E., Hand, S.C., Bowlus, R.D. and Somero, G.N. (1982) Living with water stress: evolution of osmolyte systems. Science, 217:1214-1222.
- Zentella, R., Mascorro-Gallardo, J.O., Van Dijck, P., Folch-Mallol, J., Bonini, B., Van Vaeck, C., Gaxiola, R., Covarrubias, A.A., Nieto-Sotelo, J., Thevelein, J.M. and Iturriaga G. (2000) A Selaginella lepidophylla trehalose-6-phosphate synthase complements growth and stress tolerance defects in a yeast tps1 mutant. Plant Physiology, 119: 1473-1482.

## Claims

1. A non-chimeric trehalose synthase, comprising at least trehalose-6-phosphate synthase and trehalose-6-phosphate phosphatase activity.

2. The non-chimeric trehalose synthase of claim 1, whereby said synthase comprises SEQ ID N° 2.

3. A gene encoding a non-chimeric trehalose synthase according to claim 1 or 2.

4. A gene according to claim 3, whereby said gene comprises SEQ ID N° 1.

5. A method to obtain trehalose accumulation in an organism, comprising overexpression of a non-chimeric trehalose synthase according to claim 1 or 2.

6. The method of claim 5, whereby said organism is selected from the group consisting of plants, bacteria or fungi.

7. The method of claim 6, whereby said organism is a plant.

8. The method of claim 6, whereby said organism is a lactic acid bacterium.
